# EUROPEAN PATENT SPECIFICATION

(11) **EP 3 799 082 B1**
(45) Date of publication and mention of the grant of the patent: **24.04.2024**
(21) Application number: 20183127.8
(22) Date of filing: 30.06.2020
(51) Int. Cl.: H01R 43/20, H01B 13/00, A61N 1/05, A61B 18/14

(54) **METHOD FOR CONTACTING FLEXIBLE ELECTRODES**
VERFAHREN ZUR KONTAKTIERUNG VON FLEXIBLEN ELEKTRODEN
PROCÉDÉ DE MISE EN CONTACT D'ÉLECTRODES FLEXIBLES

(30) Priority: 25.09.2019 DE 102019006709
(43) Date of publication of application: 31.03.2021
(60) Divisional application: 24159225.2
(73) Proprietor: Heraeus Medevio GmbH & Co. KG, 63450 Hanau (DE)
(72) Inventor: Jung, Markus, 63450 Hanau (DE); Musiol, Katharina, 63450 Hanau (DE)
(74) Representative: Heraeus IP

(56) References cited:
- US-A1- 2010 129 566
- US-A1- 2015 245 479
- US-A1- 2018 126 155

## Description

### FIELD OF THE INVENTION

The invention relates to a method for the electrical connection of an electrode (101) to a conductor (102) to form an electrode-conductor composite according to claim 1 and an electrode-conductor composite according to claim 13 produced according to the method according to any one of claims 1-12.

An electrode-conductor composite of this type is in particular advantageous for the use in the medical technology.

### TECHNICAL BACKGROUND

The electrical contact-connection of an electrical conductor can often represent a large challenge, in particular in the case of small dimensions. Good electrical conductivity and mechanical stability, e.g., are thereby desired even in the case of heavy use for a longer period of time. This applies in particular in the case of the electrical contact-connection of lines in medical devices. In devices, which are inserted into the human or animal body, it is desirable to use thin lines, which, however, may represent a challenge for the electrical contact-connection due to their size.

Special emphasis is placed on the reliability of medical devices, such as, e.g., pacemakers, implantable cardioverters, defibrillators, and cardiac resynchronization devices, in particular on the lowest possible material fatigue. In particular the lines and the electrical connections are exposed to high loads during operation. Due to the fact that invasive surgery is usually required to insert medical devices into the body or to remove or to replace parts thereof, a long service life of the individual components of the device is desirable in order to reduce the need for surgical procedures.

Electrical contact-connections, which are located on a flexible substrate, represent particular challenges, for example in the case of the contact-connection of conductor tracks with sensors or stimulation electrodes, which are located on flexible plastic substrates. Further difficulties also result, for example, when flexible electrodes are very thin and the leads, which connect the electrodes to the pulse generators, are dimensioned significantly larger. In many cases, the different materials in the area of the contact-connection (metal and polymers) furthermore represent a large challenge.

The contact-connection is often formed too rigidly in the prior art, so that the advantage of the flexibility gets lost. Known contact-connections are furthermore not sufficiently stable in the long term, so that the service life is not sufficiently long (fatigue breakage at the contact-connection).

It is a further disadvantage of existing contact-connections that an additional material, which is not biocompatible, is often required for the connection of the different components. These non-biocompatible materials, however, may be problematic for the use in medical devices.

In response to the bonding, as described, for example, in US20180126155A1, a contact-connection can take place without additional material. The formed contact-connection, however, is not stable in the long term and is also not suitable for all materials. The adhesion, as described, for example, in EP0612538A2, provides for a flexible connection, but an additional material is required thereby, which is generally not biocompatible and often becomes brittle after a certain period of time. Welding provides for a contact-connection without additional material, but forms a rigid, relatively large-volume contact-connection. In many cases, the welding of thin layers is demanding or impossible, because they can be thermally destroyed, in particular while attempting the contact-connection with high-melting materials, such as platinum. The soldering can be performed as welding at low temperature, but the formed contact-connections are likewise rigid, and a non-biocompatible, additional material is required.

### PREFERRED EMBODIMENTS

It is the object of the present invention to solve the above-described and further problems of the prior art. The invention provides, for example, for a contact-connection, which is stable in the long term, with flat construction in particular on flexible substrates, so that the flexibility of the entire system of lead and flexible electrode is ensured. This method can be adapted more easily to required design variations than prior art methods and provides products with improved properties, as described below. The improved contact-connection can show itself, for example, in a higher reliability, stability, and conductivity. The invention provides, for example, a contact-connection with improved breaking strength and breaking stability.

These objects are solved by means of the methods and devices described herein, in particular by means of those, which are described in the patent claims.

### DETAILED DESCRIPTION

With regard to the embodiments described herein, the elements of which "have" or "comprise" a certain feature (e.g. a material), a further embodiment is generally always considered, in which the respective element consists only of the feature, i.e. does not comprise any further components. The word "comprise" or "comprising" is used synonymously with the word "have" or "having" herein.

When an element is identified with the singular form in an embodiment, an embodiment is likewise considered, in the case of which several of these elements are present.

Unless otherwise specified or unambiguously ruled out from the context, it is generally possible and is hereby unambiguously considered that features of different embodiments can also be present in the other embodiments described herein. It is likewise generally considered that all features, which are described herein in connection with a method, are also applicable for the products and devices described herein. All of these considered combinations are not listed explicitly in all cases only for reasons of a shorter description. Technical solutions, which, as is well known, are equivalent to the features described herein, are to generally also be captured by the scope of the invention.

A first aspect of the invention relates to a method for the electrical connection of an electrode to a conductor to form an electrode-conductor composite, comprising
(i) bringing an electrode and/or a conductor into contact with a metalliferous layer,
(ii) irradiating the metalliferous layer with a laser, and
(iii) thus forming a coherent, electroconductive metal layer.

The metallic layer contains a metal or a metal oxide. Cu, Pt, Au, Ir, Pd, Ti, Ta, Fe, Nb, Ni, or alloys thereof, or further alloys, such as, for example, MP35N, are examples for suitable metals.

Platinum oxide, iridium oxide, titanium oxide, tantalum oxide, niobium oxide, copper oxide are examples for suitable metal oxides.

The metalliferous layer is preferably set up to form a coherent, electroconductive metal layer, when it is irradiated with a laser. For example particles of copper oxide, which are in a suspension, are converted into metallic copper by irradiating with a laser, so that an electroconductive coherent metal layer is formed on a substrate. Suitable copper oxide is, for example, the NanoArc copper oxide powder, which can be obtained from Alfa Aesar, with a particle dimeter of approximately 200-300 nm. This is described, for example, in Kang et al, J. Phys. Chem. C20111154823664-23670. For example infrared lasers with a wavelength of approximately 1000 nm, e.g. 1070 nm, are particularly suitable for the conversion of CuO into metallic copper. A pulse energy of 2 µJ, a pulse with of 50 ns, a pulse frequency of 300 kHz, and an irradiation surface of a diameter of 25 µm, was used thereby in the example described by *Kang et al.* It becomes evident to the person of skill in the art that other parameters, which can be determined in simple tests or which are known in the technical literary, are optionally advantageous for other materials. The values should always be selected so that the power of the laser is selected to provide for the formation of an electroconductive metal layer, without damaging the electrode, the conductor, or optionally the substrate.

In one embodiment, metal particles are fused to form a coherent electroconductive metal layer by irradiation with a laser. In one embodiment, metal particles and/or metal oxide particles are sintered to form a coherent electroconductive metal layer by irradiation with a laser.

The metalliferous layer can comprise a metal powder or a metalliferous suspension. In one embodiment, the metalliferous layer consists of a metal powder. In one embodiment, the metalliferous layer consists of a metalliferous suspension. A metalliferous suspension comprises, for example, metal particles or metal oxide particles, and a liquid, for example water. This liquid can also comprise an organic solvent, for example a mixture of poly(vinylpyrrolidone) and ethylene glycol. The liquid can comprise a dispersing agent. A dispersing agent of this type is preferably selected so that it keeps the metal particles or metal oxide particles in the colloidal state in the liquid.

In one embodiment, the electrode and/or the conductor are located on a substrate. In one embodiment, the substrate is a film. In one embodiment, the substrate comprises a plastic. In one embodiment, the substrate is flexible, for example a flexible plastic film. "Flexible" means that the substrate can be deformed significantly by manual force, without breaking or tearing, Examples for flexible substrates are plastic films, which are typically used as substrates for the production of film cables in the electronics industry. Polyester films with a thickness of 0.1-0.5 mm are examples for this. In one embodiment, the flexible substrate comprises polyurethane, polyimide, silicon, PEEK, or LCP.

The suspension can be applied to the substrate by a coating process, for example spin coating.

"Coherent" means here that a metal layer is formed, which is formed continuously in such a way that it can serve as electrical connection of two electrical components.

A conductor is an electroconductive structure, which is provided for the electrical connection of electrical or electronic components. A conductor can be, for example, a wire, for example an electrically insulated wire, or a conductor track. The wire can be part of a cable.

The conductor can be, for example, a metal wire. In some embodiments, the conductor comprises one or several of the metals Pt, Ir, Ta, Pd, Ti, Fe, Au, Ag, Mo, Nb, W, Ni, Ti, or a mixture or alloy thereof, respectively. In some embodiments, the conductor comprises the alloys MP35, PtIr10, PtIr20, 316L, 301, or nitinol. The conductor can also comprise multi-layer material systems. The conductor preferably comprises MP35, Au, Ta, Pt, Ir, or Pd. In some embodiments, a part of the conductor consists of MP35, Au, Ta, Pt, Ir, or Pd, or alloys of these metals. In some embodiments, the conductor contains less than 3%, 2%, or less than 1% of Fe.

MP35 is a curable alloy on the basis of nickel-cobalt. A variation of MP35 is described in the industrial standard ASTM F562-13. In one embodiment, MP35 is an alloy, which comprises 33 to 37% of Co, 19 to 21% of Cr, 9 to 11% of Mo, and 33 to 37% of Ni.

PtIr10 is an alloy of 88 to 92% of platinum and 8 to 12% of iridium.

PtIr20 is an alloy of 78 to 82% of platinum and 18 to 22% of iridium.

316L is an acid-resistant CrNiMo austenitic steel with approx. 17% of Cr; approx. 12% of Ni, and at least 2.0% of Mo. A variation of 316L is described in the industrial standard 10088-2. In one embodiment, 316L is an alloy, which comprises 16.5 to 18.5% of Cr; 2 to 2.5% of Mo, and 10 to 13% of Ni.

301 is a chromium nickel steel with a high corrosion resistance. A variation of 301 is described in the industrial standard DIN 1.4310. In one embodiment, 301 is an alloy, which comprises 16 to 18% of Cr and 6 to 8% of Ni.

Nitinol is a nickel-titanium alloy with shape memory with an orderly cubic crystal structure and a nickel portion of approximately 55%, wherein the remaining portion consists of titanium. Nitinol has good properties with respect to biocompatibility and corrosion resistance. Unless otherwise specified, all percentages herein are to be understood as percentage by mass (% by weight).

In one embodiment, the conductor has a spherical or a flattened form at its end. In one embodiment, the conductor is connected at this end to the metal layer. In one embodiment, the conductor is connected to the metal layer at this end by irradiation of a metalliferous layer with a laser.

An electrode is an electroconductive structure. In one embodiment, the electrode is suitable to output an electrical signal to the human body or to receive an electrical signal from the human body. An electrode can also comprise a conductor track. In one embodiment, the electrode is a conductor track, which is arranged on a substrate. In one embodiment, the conductor track is connected to a substrate. In one embodiment, the conductor track is connected directly to a substrate. In one embodiment, the electrode is a sensor or a stimulation electrode for use in the human or animal body. In one embodiment, the electrode comprises a metal or a metal alloy, for example those metals or alloys, which are described herein as being suitable for the conductor. In one embodiment, the electrode consists of a biocompatible material. A biocompatible material is a material, which is suitable for the implementation into the human or animal body. In one embodiment, the electrode comprises gold or platinum. In one embodiment, the electrode consists of gold or platinum.

In one embodiment, the electrode is set up to receive or to output an electrical signal. In one embodiment, the electrode is set up to receive an electrical signal from the human body or to output it to the human body. In some embodiments, the electrode comprises one or several of the metals Pt, Ir, Ta, Pd, Ti, Fe, Au, Mo, Nb, W, Ni, Ti, or a mixture or alloy thereof, respectively. In some embodiments, the electrode comprises the alloys MP35, PtIr20, PtIr10, PdIr10, 316L, or 301. The electrode can also comprise multi-layer material systems. In some embodiments, the electrode consists of one or several of these materials.

In one embodiment, the method comprises the steps a to c:
a. providing the electrode and the conductor on a substrate,
b. bringing the electrode and the conductor into contact with the metalliferous layer,
c. irradiating the metalliferous layer with a laser, and thus forming a coherent metal layer, which connects the electrode and the conductor in an electroconductive manner,

In step a, an electrode and a conductor are provided, which are arranged on a substrate, for example a flexible plastic film. The electrode and the conductor are preferably firmly connected to the substrate. In one embodiment, the electrode and the conductor are located on the same side of the substrate.

In step b, the electrode and the conductor are brought into contact with the metalliferous layer. For this purpose, a metalliferous layer can be applied to the substrate so that it simultaneously covers the electrode as well as the substrate at least partially. The metalliferous layer is preferably applied at that location, at which the coherent metal layer is to be created, for example along the shortest connecting line between the electrode and the conductor.

The metalliferous layer can comprise, for example, a metalliferous suspension, a paste, or a metal power, or can consist thereof. The suspension, paste, or the powder can in each case comprise a metal and/or metal oxide. Metal or metal oxide particles are present in colloidal state in a suspension, i.e. they are dispersed evenly in a liquid. A paste is a suspension, which has a particle content, which is so high that it is no longer free-flowing. A powder is a granular medium, which comprises only solid matter.

In step c, the metalliferous layer is irradiated with a laser, so that a coherent metal layer, which connects the electrode and the conductor in an electroconductive manner, is formed from the metal or metal oxide particles in the metalliferous layer. For example, metal oxide particles can thereby be reduced to form elemental metal, and/or metal particles can be fused or sintered to form a coherent layer.

In one embodiment, the method comprises steps d to g:
d. providing the conductor on a substrate,
e. bringing the conductor into contact with metalliferous layer,
f. forming the electrode on the substrate by irradiating the metalliferous layer with a laser,
g. optionally connecting the electrode to the conductor to form an electrode-conductor composite.

A conductor is hereby initially provided on a substrate, as described above. In this step, however, an electrode does not necessarily also need to be provided yet. In a further step, the conductor is brought into contact with the metalliferous layer, as described above. The metalliferous layer can comprise, for example, a metalliferous suspension, a paste, or a metal powder, or can consist thereof, as described above. In a further step, an electrode is formed by irradiation of the metalliferous layer with a laser. In one embodiment, the formation of the electrode takes place by irradiation of the metalliferous layer with a laser in such a way that an electrical connection of the electrode to the conductor is formed directly. In one embodiment, the conductor and the electrode are connected to one another in a different way. An electrode-conductor composite is formed by the electroconductive connection of the electrode to the conductor.

In a further embodiment, an electrode is initially provided on the substrate by irradiation of a metalliferous layer with a laser, and a conductor is subsequently provided and is connected to the electrode. The conductor can thereby be formed by irradiation of a metalliferous layer with a laser or can be provided in a different way. This can take place, for example, with the help of complementary structures, as described in more detail below.

In some embodiment, the electrode and/or the conductor are fixed to the substrate, so that they do not change their position during the formation of the metalliferous layer. In one embodiment, the electrode and/or the conductor are embedded into the flexible substrate by irradiation with a laser. In one embodiment, the substrate is partially melted or softened at a suitable position by irradiation of the substrate, so that the electrode and/or the conductor sink into the substrate, and are thus embedded into the substrate. In one embodiment, the electrode and/or the conductor are pressed into the substrate. In one embodiment, the substrate is softened or melted by irradiation with a laser, and the electrode and/or the conductor are subsequently pressed into the softened or melted substrate. The electrode and/or the conductor are preferably furthermore accessible to the outside, i.e. they can subsequently also be connected to one another in a conductive manner with the help of a metalliferous layer, without having to remove the substrate.

In one embodiment, the electrode and/or the conductor are fixed to the substrate by the embedding into the substrate, so that they are no longer movable relative to the substrate. The conductor and/or the electrode can, for example, be pressed or melted into the substrate.

In one embodiment, the electrode and/or the conductor are fixed to the substrate by applying an additional fixing layer. The fixing layer is preferably arranged outside of the metalliferous layer and/or of the electrical connection formed therefrom. The contact-connection area between electrode and conductor can thus be kept free from the material of the fixing layer. This fixing layer can be, for example, an adhesive, a photoresist, or a low-melting plastic. In one embodiment, the low-melting plastic has a melting temperature of less than 300°C, 200°C, or 150°C. In one embodiment, the electrode and/or the conductor is fixed to the substrate by applying an additional fixing layer prior to the irradiation of the metalliferous layer with the laser. In one embodiment, the electrode and/or the conductor is fixed by a holding device, for example a gripper or a clamping device.

In one embodiment, the conductor and/or the electrode are embedded into the substrate with the help of an injection molding process.

In one embodiment, the conductor is embedded into the substrate as described above, and an electrode is subsequently formed by irradiating a metalliferous layer. In one embodiment, the conductor as well as the electrode are embedded into the substrate as described above, and the electrode is subsequently connected to the conductor by irradiating a metalliferous layer, and thus forming a coherent metal layer.

In one embodiment, the electrode and the conductor have different diameters. This refers to the smallest diameter of the electrode or of the conductor, respectively, at that location, at which a connection between the electrode and the conductor is to take place with the help of the electroconductive metal layer. This can be, for example, the diameter of a wire or the width of a conductor track. It is possible with the help of the method according to the invention to design the geometry of the electroconductive metal layer, which is formed by irradiation of the metalliferous layer with a laser, to be virtually arbitrary. In this way, the formation of the electroconductive metal layer can take place in such a way in one embodiment that the electroconductive metal layer forms a geometrical and mechanical gradient between the diameters of the electrode and the conductor. This means that the width and/or height of the electroconductive metal layer tapers continuously between the electrode and the conductor in order to establish an even transition between the diameters of the electrode and the conductor. A gradient at the height of the electroconductive metal layer can be attained by an increasing or decreasing layer thickness of the electroconductive metal layer. This can be attained, for example, in that the laser power and/or irradiation duration is increased accordingly by variation of the movement speed or the number of the laser pass-overs of the laser at the areas, which are to have a higher layer thickness. In the alternative or in addition, the steps of bringing the electrode and/or the conductor into contact with a metalliferous layer and the irradiation of the metalliferous layer with a laser can be repeated several times, wherein a higher number of repetitions are performed in the areas with a desired higher layer thickness.

In one embodiment, a method is therefore provided, wherein the electrode and the conductor have different diameters, and the formation of an electroconductive material layer takes place in such a way that the electroconductive metal layer forms a gradient between the diameters of the electrode and of the conductor. In one embodiment, the gradient is a geometrical and/or mechanical gradient.

In one embodiment, the method furthermore comprises the removing of the metalliferous layer after the formation of the electroconductive metal layer. By the irradiation of the metalliferous layer with a laser, only a portion of the metal particles become parts of the electroconductive material layer in some cases. The remaining metal particles and optionally the carrier, which can be part of the metalliferous layer, are no longer required after the formation of the electroconductive metal layer, and can be removed. In one embodiment, the metalliferous layer is completely removed after the formation of the electroconductive metal layer. In one embodiment, the electroconductive metal layer is thereby not removed.

In one embodiment, the metalliferous layer is applied as liquid suspension, as paste, or as metal powder, as described above in more detail. The metalliferous layer can for example be applied to the substrate in such a way that it in each case partially covers the electrode and the conductor, and covers the area of the substrate located therebetween, in which the electroconductive metal layer is to form a connection between the electrode and the conductor.

In one embodiment, the electrode and/or the conductor is coated in order to improve the connection to the electroconductive metal layer. This coating can be coated, for example, with a meltable material, for example a low-melting metal. In one embodiment, the conductor is an electrically insulated wire and is coated at a non-insulated area of the wire. In one embodiment, the conductor and/or the electrode is coated with a material, which chemically reacts with the metalliferous layer. In one embodiment, the conductor and/or the electrode are coated with nanoparticles. In one embodiment, the nanoparticles are set up to chemically or physically react with the metalliferous layer, in particular with the metal or metal oxide particles contained therein. In one embodiment, the electrode and/or the conductor is coated with a material, which effects or supports the reduction of a metal oxide in the metalliferous layer.

In one embodiment, the electrode and/or the conductor is coated with a coating, which comprises a polymer. In one embodiment, the polymer is a conductive polymer, for example PEDOT, or a polymer, which comprises carbon particles or carbon nanotubes. In one embodiment, the coating comprises a metal or an alloy. In one embodiment, the metal is platinum or gold. In one embodiment, the alloy is a nickel-titanium alloy. The metal or the alloy preferably have a low melting point, for example a melting point of less than 500°C, 450°C, or 400°C. This low melting point can be attained, for example, in that the metal or the alloy are present in the form of nanoparticles. Particles with an average size diameter of less than 100 nm are referred to as nanoparticles.

In one embodiment, the electrode and/or the conductor is coated with a coating, which comprises a polymer and metal nanoparticles.

In one embodiment, the electrode and/or the conductor are provided with a structure or are roughened in order to improve the connection to the electroconductive metal layer. In one embodiment, the electrode and the conductor are provided with structures, which are in each case complementary to one another. This makes it possible that the electrode and the conductor can be connected to one another in a positive manner. In one embodiment, the electrode and the conductor are connected to one another in a positive manner as well as in a non-positive manner. In one embodiment, the electrode and the conductor are connected to one another in a positive manner as well as by means of a substance-to-substance bond. In one embodiment, the substance-to-substance bond of the electrode to the conductor comprises the irradiation of a metalliferous layer with a laser, and thus formation of a coherent electroconductive metal layer, which connects the electrode to the conductor. In one embodiment, the substance-to-substance bond of the electrode to the conductor comprises the cold welding (press fit) of the electrode to the conductor. In one embodiment, the conductor and the electrode are connected to one another by pressing.

In one embodiment, an electrically insulating layer is additionally applied to the electroconductive metal layer. In one embodiment, the electroconductive metal layer is covered completely by the electrically insulating layer. In one embodiment, the electrically insulating layer comprises a polymer. In one embodiment, the polymer is selected from the group consisting of silicon, polyethylene, polyurethane, polyimide, polyamide, PEEK, fluorinated plastics. Fluorinated plastics are, for example, ETFE, PTFE, PFA, PVDF, or FEP.

A further aspect of the invention relates to an electrode-conductor composite, which is produced or can be produced according to any one of the above-described methods. An electrode-conductor composite comprises an electrode, which is connected to a conductor in an electroconductive manner. In one embodiment, the electrode-conductor composite is electrical medical device or a part of a device of this type. The electrical medical device can be, e.g., a lead, pulse generator, pacemaker, cardiac resynchronization device, catheter, sensor, or stimulator. Leads are electrical lines, which can be used, for example, in medical applications, such as neuromodulation, cardiac stimulation, deep brain stimulation, spinal cord stimulation, peripheral nerve stimulation, or gastric stimulation. Examples for catheters according to the invention are those, which are set up for the electrophysiological mapping or the ablation of tissue. In one embodiment, the lead is set up and/or intended to be connected to a generator of an active implantable device. A lead of the invention can also be used in a medical device in order to receive an electrical signal. A stimulator is a medical device, which can have a physiological effect by outputting an electrical signal to the body of a living being. For example, a neurostimulator can effect an electrical signal in the nerve cell (e.g. an action potential) by outputting an electrical signal to a nerve cell. A further embodiment relates to a microelectrode or a microelectrode array, which comprises an electrode-conductor composite described herein.

The invention will be described in more detail below on the basis of the figures in several examples. These examples serve to further illustrate the invention. It is not intended that these examples are to limit the scope of protection of this patent application.

### EXAMPLES

### LIST OF THE FIGURES

Fig. 1 describes a first example for a method according to the invention.
Fig. 2 describes a second example for a method according to the invention.
Fig. 3 describes a third example for a method according to the invention.

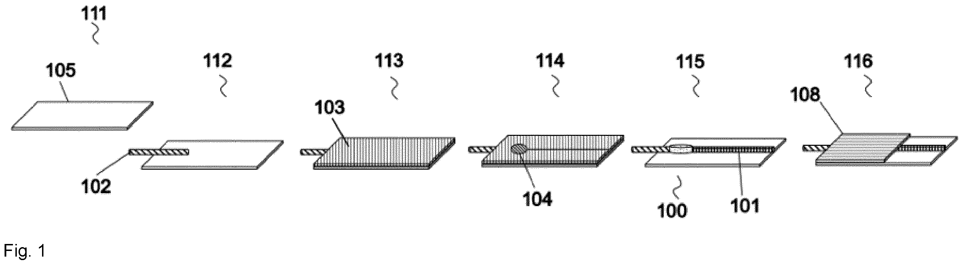

Fig. 1 describes a first method. In a method step 111, a substrate 105 is initially provided. In a step 112, a conductor 102 is arranged on the substrate, The conductor 102 can be fixed to the substrate 105 temporarily or permanently. The conductor 102 can be a wire, for example. The conductor 102 can be pressed into the substrate and/or can be partially melted into the substrate in order to fix the conductor 102 to the substrate 105. In step 113, a metalliferous layer is applied to the substrate 105, so that it at least partially covers the conductor 102. The metalliferous layer 103 covers the substrate 105 and the conductor 102 at least at that location, at which a coherent conductive metal layer 104 is to be formed. In step 114, the metalliferous layer 103 is irradiated at this location with a laser (not shown), so that the coherent conductive metal layer 104 forms. An electrode 101 is formed from the metal layer 104 in this example, namely in such a way that the electrode 101 is connected to the conductor 102 in an electroconductive manner, thus forms an electrode-conductor composite on the substrate 105. In step 115, the remaining metalliferous layer 103 is removed. What remains is the electrode-conductor composite on the substrate 105. In step 116, the resulting electrode-conductor composite is covered with an electrically insulating layer 108. The metal layer 104 can be protected against damages thereby. The step 116 is optional and can also be omitted.

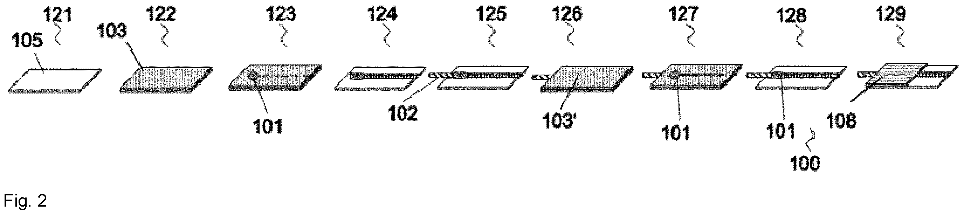

Fig. 2 describes a second method. In step 121, a substrate 105 is provided. In step 122, a metalliferous layer 103 is applied to the substrate 105. In step 123, the metalliferous layer 103 is irradiated with a laser (not shown), so that an electrode 101 made of metal forms on the substrate 105. In step 124, the remaining metalliferous layer 103 is removed. In step 125, a conductor 102 is provided and is brought into contact with the electrode 101. In step 126, the substrate 105, the conductor 102, and the electrode 101 are covered with a further metalliferous layer 103'. In step 127, the further metalliferous layer 103' is irradiated with a laser, so that a further coherent electroconductive metal layer forms, which connects the electrode 101 to the conductor 102 in an electroconductive manner. In step 128, the remaining further metalliferous layer is removed. What remains is an electrode-conductor composite, in which the conductor 102 is connected to the electrode 101 in an electroconductive manner. In the optional step 129, an electrically insulating layer 108 is applied to the further electroconductive metal layer 103'. In the example of Fig. 2, the electrode 101 as well as the electroconductive metal layer, which connects the electrode to the conductor 102, is in each case formed by irradiation of a metalliferous layer 103, 103' with a laser. The two metalliferous layers 103, 103' can be identical or different.

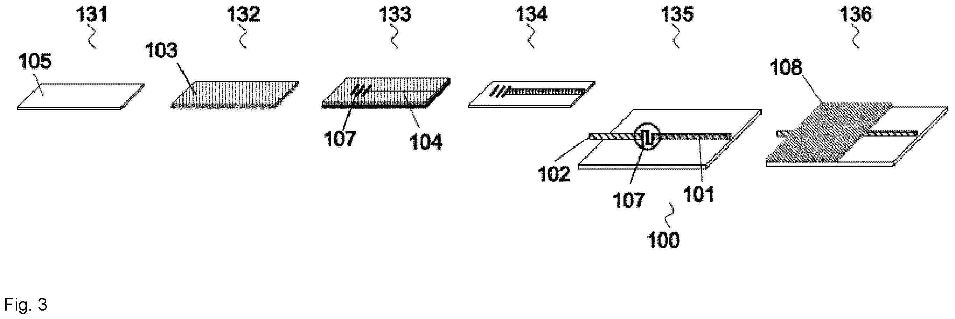

Fig. 3 describes a third method. In step 131, a substrate 105 is provided. In step 132, the substrate 105 is covered with a metalliferous layer 103 and is thus brought into contact with the substrate 105. In step 133, the metalliferous layer 103 is irradiated with a laser, so that an electroconductive, coherent metal layer 104 forms on the substrate 105. The laser is thereby guided in such a way that the metal layer 104 forms an electrode 101 with a structure 107 at the end of the electrode 101. In step 134, the excess portion of the metalliferous layer 103 is removed. In step 135, a conductor 102 is provided, which has a structure 107`, which is complementary to the structure 107 of the electrode 101, so that the two structures 107, 107'engage with one another and can be connected to one another in a positive manner. By connecting the electrode 101 to the conductor 102 with the help of the structures 107, 107`, an electrode-conductor composite 100 is formed. In a further, optional step 136, the structures 107, 107` are covered with electrically insulating layer 108.

## Claims

1. A method for forming an electrical connection of an electrode (101) to a conductor (102) to form an electrode-conductor composite (100), comprising
(i) bringing an electrode (101) and/or a conductor (102) into contact with a metalliferous layer (103) that comprises a metal powder or a metalliferous suspension, or consists thereof;
(ii) irradiating the metalliferous layer (103) with a laser; and
(iii) thus forming a coherent, electroconductive metal layer (104).

2. The method according to claim 1, comprising the steps a to c,
a. providing the electrode (101) and the conductor (102) on a substrate (105),
b. bringing the electrode (101) and the conductor (102) into contact with the metalliferous layer (103),
c. irradiating the metalliferous layer (103) with a laser, and thus forming a coherent metal layer (104), which connects the electrode (101) and the conductor (102) in an electroconductive manner,
or the steps d to g,
d. providing the conductor (102) on a substrate (105),
e. bringing the conductor (102) into contact with the metalliferous layer (103),
f. forming the electrode (101) on the substrate (105) by irradiating the metalliferous layer (103) with a laser,
g. optionally connecting the electrode (101) to the conductor (102) to form an electrode-conductor composite (100).

3. The method according to any one of the preceding claims, wherein the forming of the coherent, electroconductive metal layer (104) takes place by laser sintering of metal particles, or by the laser-induced reduction of a metal oxide.

4. The method according to any one of the preceding claims, wherein the electrode (101) and/or the conductor (102) are embedded into the flexible substrate (105) by irradiation with the laser.

5. The method according to any one of the preceding claims, wherein the electrode (101) and the conductor (102) have different diameters, and the formation of an electroconductive metal layer (104) takes place in such a way that the electroconductive metal layer (104) forms a gradient between the diameters of the electrode (101) and the conductor (102).

6. The method according to any one of the preceding claims, further comprising
h. removing the metalliferous layer (103) after the formation of the electroconductive metal layer (104).

7. The method according to any one of the preceding claims, wherein the metalliferous layer (103) is applied as liquid suspension, as paste, as solid coating, or as metal powder.

8. The method according to any one of claims 3 to 7, wherein the electrode (101) and/or the conductor (102) are fixed to the substrate (105) by applying an additional fixing layer (106) prior to the irradiation of the metalliferous layer (103) with a laser.

9. The method according to any one of the preceding claims, wherein the electrode (101) and/or the conductor (102) are coated in order to improve the connection to the electroconductive metal layer (104).

10. The method according to any one of the preceding claims, wherein the electrode (101) and/or the conductor (102) are provided with a structure (107) or are roughened in order to improve the connection to the electroconductive metal layer (104), wherein the electrode (101) and the conductor (102) are preferably provided with structures (107, 107'), which are complementary to one another.

11. The method according to any one of claims 2 to 10, wherein the method comprises steps d to g of claim 2, and wherein the electrode (101) and the conductor (102) are connected to one another by pressing.

12. The method according to any one of the preceding claims, further comprising applying an electrically insulating layer (108) to the electroconductive metal layer (104).

13. An electrode-conductor composite (100), which is produced according to a method according to any one of the preceding claims.

## Patentansprüche

1. Verfahren zum Bilden einer elektrischen Verbindung einer Elektrode (101) mit einem Leiter (102), um einen Elektrodenleiterverbundstoff (100) zu bilden, umfassend
(i) Inkontaktbringen einer Elektrode (101) und/oder eines Leiters (102) mit einer metallhaltigen Schicht (103), die ein Metallpulver oder eine metallhaltige Suspension umfasst oder daraus besteht;
(ii) Bestrahlen der metallhaltigen Schicht (103) mit einem Laser; und
(iii) dadurch Bilden einer kohärenten, elektrisch leitfähigen Metallschicht (104).

2. Verfahren nach Anspruch 1, umfassend die Schritte a bis c,
a. Bereitstellen der Elektrode (101) und des Leiters (102) auf einem Substrat (105),
b. Inkontaktbringen der Elektrode (101) und des Leiters (02) mit der metallhaltigen Schicht (103),
c. Bestrahlen der metallhaltigen Schicht (103) mit einem Laser und dadurch Bilden einer kohärenten Metallschicht (104), die die Elektrode (101) und den Leiter (102) elektrisch leitend verbindet,
oder die Schritte d bis g,
d. Bereitstellen des Leiters (102) auf einem Substrat (105),
e. Inkontaktbringen des Leiters (102) mit der metallhaltigen Schicht (103),
f. Bilden der Elektrode (101) auf dem Substrat (105) durch Bestrahlen der metallhaltigen Schicht (103) mit einem Laser,
g. optional Verbinden der Elektrode (101) mit dem Leiter (102), um einen Elektrodenleiterverbundstoff (100) zu bilden.

3. Verfahren nach einem der vorstehenden Ansprüche, wobei das Bilden der kohärenten, elektrisch leitfähigen Metallschicht (104) durch Lasersintern von Metallpartikeln oder durch die laserinduzierte Reduktion eines Metalloxids erfolgt.

4. Verfahren nach einem der vorstehenden Ansprüche, wobei die Elektrode (101) und/oder der Leiter (102) durch Bestrahlung mit dem Laser in das flexible Substrat (105) eingebettet sind.

5. Verfahren nach einem der vorstehenden Ansprüche, wobei die Elektrode (101) und der Leiter (102) unterschiedliche Durchmesser aufweisen und die Bildung einer elektrisch leitfähigen Metallschicht (104) derart erfolgt, dass die elektrisch leitfähige Metallschicht (104) einen Gradienten zwischen den Durchmessern der Elektrode (101) und des Leiters (102) bildet.

6. Verfahren nach einem der vorstehenden Ansprüche, ferner umfassend h. Entfernen der metallhaltigen Schicht (103) nach der Bildung der elektrisch leitfähigen Metallschicht (104).

7. Verfahren nach einem der vorstehenden Ansprüche, wobei die metallhaltige Schicht (103) als flüssige Suspension, als Paste, als feste Beschichtung oder als Metallpulver aufgebracht wird.

8. Verfahren nach einem der Ansprüche 3 bis 7, wobei die Elektrode (101) und/oder der Leiter (102) durch Aufbringen einer zusätzlichen Befestigungsschicht (106) vor der Bestrahlung der metallhaltigen Schicht (103) mit einem Laser an dem Substrat (105) befestigt werden.

9. Verfahren nach einem der vorstehenden Ansprüche, wobei die Elektrode (101) und/oder der Leiter (102) beschichtet sind, um die Verbindung mit der elektrisch leitfähigen Metallschicht (104) zu verbessern.

10. Verfahren nach einem der vorstehenden Ansprüche, wobei die Elektrode (101) und/oder der Leiter (102) mit einer Struktur (107) versehen oder aufgeraut sind, um die Verbindung mit der elektrisch leitfähigen Metallschicht (104) zu verbessern, wobei die Elektrode (101) und der Leiter (102) vorzugsweise mit Strukturen (107, 107') versehen sind, die komplementär zueinander sind.

11. Verfahren nach einem der Ansprüche 2 bis 10, wobei das Verfahren die Schritte d bis g nach Anspruch 2 umfasst, und wobei die Elektrode (101) und der Leiter (102) durch Pressen miteinander verbunden sind.

12. Verfahren nach einem der vorstehenden Ansprüche, ferner umfassend das Aufbringen einer elektrisch isolierenden Schicht (108) auf die elektrisch leitfähige Metallschicht (104).

13. Elektrodenleiterverbundstoff (100), der gemäß einem Verfahren nach einem der vorstehenden Ansprüche hergestellt ist.

## Revendications

1. Procédé permettant de former une connexion électrique d'une électrode (101) à un conducteur (102) pour former un composite électrode-conducteur (100), comprenant
(i) le fait d'amener une électrode (101) et/ou un conducteur (102) en contact avec une couche métallifère (103) qui comprend une poudre métallique ou une suspension métallifère, ou est constitué de celle-ci ;
(ii) l'irradiation de la couche métallifère (103) avec un laser ; et
(iii) la formation ainsi d'une couche métallique (104) cohérente électroconductrice.

2. Procédé selon la revendication 1, comprenant les étapes a à c,
a. la fourniture de l'électrode (101) et du conducteur (102) sur un substrat (105),
b. le fait d'amener l'électrode (101) et le conducteur (102) en contact avec la couche métallifère (103),
c. l'irradiation de la couche métallifère (103) avec un laser, et la formation ainsi d'une couche métallique (104) cohérente, qui connecte l'électrode (101) et le conducteur (102) d'une manière électroconductrice,
ou les étapes d à g,
d. la fourniture du conducteur (102) sur un substrat (105),
e. le fait d'amener le conducteur (102) en contact avec la couche métallifère (103),
f. la formation de l'électrode (101) sur le substrat (105) par irradiation de la couche métallifère (103) avec un laser,
g. la connexion facultative de l'électrode (101) au conducteur (102) pour former un composite électrode-conducteur (100).

3. Procédé selon l'une quelconque des revendications précédentes, dans lequel la formation de la couche métallique (104) cohérente électroconductrice se déroule par frittage laser de particules métalliques, ou par la réduction induite par laser d'un oxyde métallique.

4. Procédé selon l'une quelconque des revendications précédentes, dans lequel l'électrode (101) et/ou le conducteur (102) sont intégrés dans le substrat flexible (105) par irradiation avec le laser.

5. Procédé selon l'une quelconque des revendications précédentes, dans lequel l'électrode (101) et le conducteur (102) ont des diamètres différents, et la formation d'une couche métallique (104) électroconductrice se déroule d'une manière telle que la couche métallique (104) électroconductrice forme un gradient entre les diamètres de l'électrode (101) et du conducteur (102).

6. Procédé selon l'une quelconque des revendications précédentes, comprenant en outre
h. le retrait de la couche métallifère (103) après la formation de la couche métallique (104) électroconductrice.

7. Procédé selon l'une quelconque des revendications précédentes, dans lequel la couche métallifère (103) est appliquée en tant que suspension liquide, en tant que pâte, ou en tant que revêtement solide, ou en tant que poudre métallique.

8. Procédé selon l'une quelconque des revendications 3 à 7, dans lequel l'électrode (101) et/ou le conducteur (102) sont fixés au substrat (105) en appliquant une couche de fixation supplémentaire (106) avant l'irradiation de la couche métallifère (103) avec un laser.

9. Procédé selon l'une quelconque des revendications précédentes, dans lequel l'électrode (101) et/ou le conducteur (102) sont revêtus afin d'améliorer la connexion à la couche métallique (104) électroconductrice.

10. Procédé selon l'une quelconque des revendications précédentes, dans lequel l'électrode (101) et/ou le conducteur (102) sont pourvus d'une structure (107) ou sont rendus rugueux afin d'améliorer la connexion à la couche métallique (104) électroconductrice, dans lequel l'électrode (101) et le conducteur (102) sont de préférence pourvus de structures (107, 107'), qui sont complémentaires l'une à l'autre.

11. Procédé selon l'une quelconque des revendications 2 à 10, dans lequel le procédé comprend les étapes d à g de la revendication 2, et dans lequel l'électrode (101) et le conducteur (102) sont connectés l'un à l'autre par pressage.

12. Procédé selon l'une quelconque des revendications précédentes, comprenant en outre l'application d'une couche électriquement isolante (108) à la couche métallique (104) électroconductrice.

13. Composite électrode-conducteur (100), qui est produit selon un procédé selon l'une quelconque des revendications précédentes.
